# EUROPÄISCHE PATENTSCHRIFT

(11) **EP 3 468 642 B1**
(45) Veröffentlichungstag und Bekanntmachung des Hinweises auf die Patenterteilung: **23.03.2022**
(21) Anmeldenummer: 17732788.9
(22) Anmeldetag: 07.06.2017
(51) Int. Cl.: A61M 5/142, A61M 5/158, A61J 1/20, A61M 5/168

(54) **DOSIERGERÄT UND INJEKTIONSVORRICHTUNG**
DOSING APPARATUS AND INJECTION DEVICE
APPAREIL DE DOSAGE ET DISPOSITIF D'INJECTION

(30) Priorität: 08.06.2016 EP 16173561
(43) Veröffentlichungstag der Anmeldung: 17.04.2019
(73) Patentinhaber: SHL Medical AG, 6300 Zug (CH)
(72) Erfinder: WEIBEL, Ludwig Daniel, 9104 Waldstatt (CH); WYLER, Samuel, 9030 Abtwil (CH); EGLOFF, Christoph, 8224 Löhningen (CH)
(86) Internationale Anmeldenummer: PCT/EP2017/063750
(87) Internationale Veröffentlichungsnummer: WO 2017/211851

(56) Entgegenhaltungen:
- WO-A1-2009/098306
- WO-A1-2014/090745
- WO-A2-2012/103428
- CN-A- 101 687 075
- US-A1- 2011 073 620

## Beschreibung

Die vorliegende Erfindung betrifft ein Dosiergerät zur insbesondere subkutanen Abgabe eines Fluids gemäss den Oberbegriffen der unabhängigen Ansprüche. Genauer betrifft die vorliegende Erfindung ein derartiges Dosiergerät welches alternativ oder zusätzlich zur subkutanen Abgabe zur intradermalen, intramuskulären oder intraperitonealen Abgabe eines Fluids geeignet ist.

Bei der Verabreichung flüssiger Formulierungen pharmazeutischer Wirkstoffe ist es in den meisten Fällen erforderlich, wohl definierte Volumina abzugeben. Häufig müssen Arzneimittel dabei in den Körper eines Patienten injiziert werden. Zur parenteralen Injektion kommen dabei Injektionsspritzen, Arzneimittelstifte (sog. Pens) oder Arzneimittelpumpen zum Einsatz. Insbesondere bei Präparaten, die über einen längeren Zeitraum und/oder nach einem genau vorgegebenen Schema verabreicht werden müssen, werden Spritzen und Pens immer mehr von Arzneimittelpumpen abgelöst. So müssen beispielsweise Patienten, die unter Diabetes leiden, sich oft mehrmals täglich unter stark wechselnden Bedingungen selbst eine Insulindosis verabreichen. Gerade bei einer solchen Anwendung stellt der Einsatz einer Arzneimittelpumpe eine bedeutende Vereinfachung für den Patienten dar, da die Handhabung einer solchen Pumpe eine deutlich geringere Einschränkung bedeutet als diejenige einer Spritze oder eines Pens. Arzneimittelpumpen sind je nach Ausführung dazu geeignet, über einen längeren Zeitraum am Körper eines Patienten angebracht zu werden und ein Präparat kontinuierlich und/oder nach einem individuell vorgegebenen Schema zu verabreichen. Derartige Arzneimittelpumpen umfassen typischerweise einen Behälter für die flüssige Formulierung sowie eine Fördervorrichtung, welche das Arzneimittel zu einem Anschluss der Vorrichtung oder zu einem Injektionssystem fördert. Ein solches Injektionssystem kann zum Setzen einer Verweilkanüle geeignet sein, die während des gesamten Verabreichungszeitraums im Körper des Patienten verbleibt.

So zeigt die WO 2014/191038 A1 ein Dosiergerät zur subkutanen Abgabe eins Fluids. Das besagte Gerät umfasst eine Injektionsvorrichtung mit einer flexiblen Verweilkanüle, wobei eine Einstechkanüle koaxial im Inneren der Verweilkanüle verläuft, um diese beim Setzen zu stützten. Nach dem Setzvorgang wird die Einstechkanüle aus der Verweilkanüle zurückgezogen, um die Abgabe für den Patienten weniger unangenehm zu machen. Neben der Injektionsvorrichtung umfasst das besagte Gerät ferner einen kollabierbaren Behälter mit einem Innenraum zur Aufnahme des Fluids. Der Behälter ist über ein Verschlussstück an einer Fördervorrichtung angeschlossen, welche als ventillose Taumelkolbenpumpe ausgebildet ist. Das Dosiergerät ermöglicht eine vollautomatische subkutane Abgabe eines Fluids an einen Patienten.

Allerdings ist das Gerät in Bezug auf seine Mechanik vergleichsweise komplex ausgeführt. So bilden die Fördervorrichtung und die Injektionsvorrichtung zwei vollständig unabhängige Einheiten. Diese sind jeweils aus einer grossen Anzahl von Einzelteilen zusammengesetzt, was die Herstellung kostenintensiv macht und auf Kosten der Zuverlässigkeit in der Anwendung geht.

Die WO 2009/098306 A1 bezieht sich auf eine Injektionsvorrichtung, bei der das Setzen einer Verweilkanüle mit Hilfe einer Einstechkanüle über eine Kulissenführung erzielt wird. Zwar sind die Bewegungen der Verweilkanüle und der Einstechkanüle über die Kulissenführung in konstruktiv einfacher und zuverlässiger Weise mechanisch gekoppelt, allerdings ist die beschriebene Vorrichtung noch immer vergleichsweise umständlich in der Anwendung.

Einerseits muss die eigentliche Injektionsvorrichtung nach dem Setzen der Verweilkanüle von einer an der Haut eines Patienten angebrachten Grundplatte entfernt werden. Andererseits muss manuell eine Fluidverbindung zwischen der Verweilkanüle und Fluidquelle hergestellt werden. Eine automatische Benutzung der Vorrichtung ist damit nicht möglich. Auch konstruktiv weist die besagte Vorrichtung einige Nachteile auf. So sind an der Grundplatte spezielle Rastmittel für die Verweilkanüle erforderlich. Darüber hinaus weist die Injektionsvorrichtung eine vergleichsweise grosse Bauhöhe auf.

Die CN101687075 A zeigt Systeme, Verfahren und Vorrichtungen zum Abgeben von therapeutischer Flüssigkeit an den Körper eines Patienten. Die Vorrichtung umfasst eine Abgabeeinheit mit einem Antriebsmechanismus, einem Pumpmechanismus, einem Heilfluid enthaltenden Reservoir und einer Auslassöffnung. Der Antriebsmechanismus bewirkt, dass der Pumpmechanismus therapeutische Flüssigkeit innerhalb des Reservoirs positiv verdrängt, um therapeutische Flüssigkeit über die Auslassöffnung an den Körper des Patienten abzugeben.

Die WO 2014/090745 A1 zeigt eine medizinische Pumpe zum Fördern einer Flüssigkeit und ein Verfahren zum Betreiben der medizinischen Pumpe. Die medizinische Pumpe arbeitet nach dem Prinzip einer Doppelkolbenpumpe.

Die US2011/073620 A1 offenbart eine verdreht-translatorische Pumpenkartusche zum Unterfüllen elektronischer Geräte mit viskosen Flüssigkeiten. Sie umfasst eine Polymerhülle mit beweglichen Kernen, die von jedem Ende eingeführt werden. Die Polymerhülle enthält zwei senkrechte Durchgänge entlang der Oberseite zum Verbinden einer Hauptfluidkammer, einer Hauptfluidversorgungskammer und eines Fluidreservoirs. Diese senkrechten Durchgänge und entsprechenden Verbindungsdetails sind senkrecht zur Achse der Polymerhülle angeordnet, die den Pumpenkartuschenhohlraum umfasst. Unmittelbar unterhalb befindet sich an einer anderen relativen Position auch senkrecht zur Achse des Pumpenkartuschenhohlraums ein Austritts-Senkrechtdurchgang zum Extrudieren von Flüssigkeit. Fluid bewegt sich innerhalb der Kartusche durch Translation, nach dem Prinzip einer Doppelkolbenpumpe.

Es ist daher die Aufgabe der vorliegenden Erfindung, die Nachteile im Stand der Technik zu überwinden.

Insbesondere ist es eine Aufgabe der vorliegenden Erfindung, ein vielfältig anwendbares und konstruktiv einfaches Dosiergerät zur insbesondere subkutanen Abgabe eines Fluids optional aufweisend eine Injektionsvorrichtung zur subkutanen Abgabe eines Fluids zu schaffen. Dieses soll in Bezug auf die Mechanik einfach und damit kostengünstig herstellbar sein. Darüber hinaus soll es bei der Anwendung benutzerfreundlich sein, eine automatische Verwendung ermöglichen und eine hohe Zuverlässigkeit aufweisen.

Die vorliegende Erfindung ist alternativ oder zusätzlich zur subkutanen Abgabe zur intradermalen, intramuskulären oder intraperitonealen Abgabe eines Fluids geeignet.

Diese Aufgaben werden durch ein Dosiergerät gelöst, welches die Merkmale im unabhängigen Anspruch 1 aufweist.

Eine optionale Injektionsvorrichtung zur subkutanen Abgabe eines Fluids gemäss der Offenbarung umfasst eine Einstechkanüle und eine Verweilkanüle. In einer Ausgangsposition verläuft ein distaler Endbereich der Einstechkanüle koaxial im Inneren der Verweilkanüle. Die Vorrichtung umfasst einen ersten und einen zweiten verschiebbar gelagerten Läufer. Der erste Läufer ist mit der Einstechkanüle und der zweite Läufer mit der Verweilkanüle verbunden. Die optionale Injektionsvorrichtung umfasst ferner ein über einen vordefinierten Steuerbereich bewegbares Steuerelement, das zum Verschieben des ersten Läufers und des zweiten Läufers mit diesen in Wirkverbindung bringbar ist. Das Steuerelement ist derart ausgebildet, dass es in einem ersten Teil des Steuerbereichs ein gleichgerichtetes, insbesondere simultanes, Verschieben der beiden Läufer, und damit ein Setzen der Verweilkanüle bewirkt. Das Steuerelement ist darüber hinaus derart ausgebildet, dass es in einem zweiten Teil des Steuerbereiches ein Blockieren des zweiten Läufers, und damit ein Halten der Verweilkanüle in einer Verweilposition, sowie ein Zurückverschieben des ersten Läufers, und damit ein Zurückziehen der Einstechkanüle aus dem distalen Endbereich der Verweilkanüle in eine Endposition, bewirkt.

Unabhängig davon betrifft die vorliegende Offenbarung auch eine derartige Injektionsvorrichtung welche alternativ oder zusätzlich zur subkutanen Abgabe zur intradermalen, intramuskulären oder intraperitonealen Abgabe eines Fluids geeignet ist.

Durch diese mechanische Ausführung der optionalen Injektionsvorrichtung kann ein durch die Einstechkanüle gestütztes Setzen der Verweilkanüle gefolgt von einem Zurückziehen der Einstechkanüle zuverlässig bewirkt werden. Der Mechanismus kommt mit einer geringen Anzahl von Einzelteilen aus, wodurch dieser kostengünstig herstellbar und zuverlässig in seiner Anwendung ist.

Die Läufer können über eine Führungsvorrichtung, vorzugsweise über eine gemeinsame Führungsvorrichtung, insbesondere über eine Linearführung verschiebbar gelagert sein. Der Einsatz einer gemeinsamen Führungsvorrichtung ermöglicht es, die Anzahl der Einzelteile der Injektionsvorrichtung weiter zu reduzieren. Insbesondere eine Linearführung ermöglicht eine Vereinfachung des Mechanismus.

So können die Läufer über eine gemeinsame Linearführung verschiebbar gelagert sein und die Linearführung in einer Setzrichtung parallel zu den distalen Endbereichen der Einstechkanüle und der Verweilkanüle ausgerichtet sein. Damit gibt die Linearführung nicht nur die Richtung des Verschiebens des ersten und des zweiten Läufers vor, sondern auch die Setzrichtung der Injektionsvorrichtung. Diese kann damit vorgängig definiert und der jeweiligen Anwendung der Vorrichtung angepasst werden. So ist es beispielsweise möglich, die Injektionsvorrichtung derart auszulegen, dass die Verweilkanüle im Wesentlichen senkrecht unter die Haut des Patienten gesetzt wird.

Das Steuerelement kann in einer Richtung, insbesondere im Wesentlichen, senkrecht zu derjenigen der Linearführung bewegbar, insbesondere verschiebbar, gelagert sein. Dies ermöglicht eine optimale Kraftübertragung vom Steuerelement auf den ersten und den zweiten Läufer in beide Richtungen.

Das Steuerelement kann als verschiebbarer Kurventräger ausgebildet sein. Ein Kurventräger hat den Vorteil, dass mit ihm nahezu jede Bewegung, die über eine stetig-differenzierbare Funktion darstellbar ist, vorgegeben werden kann.

Das Steuerelement kann als beidseitig auf die Läufer wirkendes Paar von deckungsgleichen, verschiebbaren Kurventrägern ausgebildet sein. Dies ermöglicht es, die Kraft besonders wirkungsvoll vom Steuerelement auf die Läufer zu übertragen. Da die Kraftübertragung beidseitig erfolgt, kann verhindert werden, dass gleichzeitig ein Drehmoment auf die Läufer übertragen wird.

Damit kann ein Verkanten derselben in einer Führungsvorrichtung vermieden werden.

Das Steuerelement kann einen ersten und einen zweiten Abschnitt umfassen. Der erste Abschnitt kann ein gleichgerichtetes, insbesondere simultanes, Verschieben der beiden Läufer, und damit ein Setzen der Verweilkanüle, bewirken. Der zweite Abschnitt kann ein Blockieren des zweiten Läufers, und damit ein Halten der Verweilkanüle in einer Verweilposition, sowie ein Zurückziehen des ersten Läufers, und damit ein Zurückziehen der Einstechkanüle aus dem distalen Endbereich der Verweilkanüle in eine Endposition, bewirken. Dabei können insbesondere unterschiedliche Seiten des zweiten Abschnittes auf den ersten und auf den zweiten Läufer wirken. Durch die Unterteilung des Steuerelementes in zwei Abschnitte kann dieses in Bezug auf seine Geometrie besonders einfach ausgestaltet werden. Die Bewegung des ersten Läufers und des zweiten Läufers kann durch Kurvenflächen am Steuerelement vorgegeben sein.

Das Steuerelement kann über ein Federelement, insbesondere über eine Schraubenfeder, vorgespannt sein. Dies ermöglicht es, auf konstruktiv einfache Weise ein selbstständiges Verschieben des Steuerelementes zu bewirken, ohne dass dazu noch ein gesonderter Antrieb erforderlich wäre.

Allerdings kann das Steuerelement auch über ein Schraubengetriebe, insbesondere über einen Spindeltrieb, bewegbar sein. Dadurch kann das Steuerelement leicht durch einen Drehantrieb mit der erforderlichen Untersetzung bewegt werden.

Allerdings kann das Steuerelement auch über eine Kurventrommel bewegbar und/oder gegen eine Federvorspannung haltbar sein. Im Gegensatz zu einem Schraubengetriebe ermöglicht eine Kurventrommel die Implementierung komplexerer Bewegungsabläufe des Steuerelementes. So ist es beispielsweise denkbar, dass sich die Kurventrommel erst um einen vordefinierten Winkel drehen muss, bis das Steuerelement freigegeben und durch die Federvorspannung bewegbar ist. Ähnlich wie beim Steuerelement selbst sind mit einer Kurventrommel prinzipiell alle Bewegungen realisierbar, die durch eine stetig-differenzierbare Funktion beschreibbar sind. So ist es beispielsweise denkbar, dass sich das Steuerelement, angetrieben durch die Kurventrommel in unterschiedlichen Teilen des Steuerbereiches in verschiedene Richtungen oder unterschiedlich schnell bewegt.

Der zweite Läufer kann als Halteblech ausgeführt sein. Ein Halteblech hat den Vorteil, dass es im Vergleich zu einem Spritzgussteil einfacher fertigbar ist. Zudem weist es bei gleicher Wandstärke eine grössere mechanische Belastbarkeit auf. Daher kann es vergleichsweise flach ausgeführt werden, wodurch die Gesamtbauhöhe der Injektionsvorrichtung reduziert werden kann. Ausserdem kann das Halteblech, falls erwünscht, eine gewisse Federwirkung zeigen.

Ein Übergang von der Einstechkanüle zur Verweilkanüle kann mit einem Dichtungselement abgedichtet sein. Das Dichtungselement kann in der Verweilposition gequetscht werden, insbesondre durch einen Wandabschnitt eines Dosiergerätes und den zweiten Läufer. Damit kann ein besonders fluiddichter Übergang von der Einstechkanüle zur Verweilkanüle erzielt werden. Es versteht sich von selbst, dass diese Ausführung der Injektionsvorrichtung auch unabhängig von anderen Merkmalen der Vorrichtung realisiert werden kann.

Die Verweilkanüle kann einen Flansch oder einen flanschartigen Bereich mit erhöhter Wandstärke aufweisen. Der Flansch, bzw. der flanschartige Bereich, kann in der Verweilposition gequetscht werden, insbesondre durch einen Wandabschnitt eines Dosiergerätes und den zweiten Läufer. Dadurch kann ein Halten der Verweilkanüle erzielt werden. Dies kann insbesondere von Bedeutung sein, wenn das Dosiergerät von der Hauteines Patienten entfernt und die Verweilkanüle u.a. aus dessen Haut herausgezogen wird. Ein unbeabsichtigtes Steckenbleiben der Verweilkanüle kann damit nämlich vermieden werden.

Die Einstechkanüle kann als Hohlnadel, ausgebildet sein. Diese Ausführung ermöglicht es, dass die Einstechkanüle gleichzeitig eine Leitungsfunktion und eine Penetrationsfunktion wahrnimmt. Dabei kann die Einstechkanüle einstückig durchgehend, insbesondere aus Stahl, ausgebildet sein. Entsprechend können Dichtungsprobleme zwischen verschiedenen Leitungsabschnitten vermieden werden. Der proximale Endbereich der Einstechkanüle kann ortsfest und starr angeordnet sein. Dadurch kann eine fluiddichte Verbindung, beispielsweise mit einer Fördervorrichtung, leicht gewährleistet werden. Insbesondere kann der distale Endbereich der Einstechkanüle in der Endposition im proximalen Endbereich der Verweilkanüle verbleiben. Dies ermöglicht einen Fluidübergang von der Einstechkanüle zur Verweilkanüle auf konstruktiv einfache Weise. Insbesondere kann in der Endposition durch die Einstechkanüle und die Verweilkanüle ein Fluidpfad zur insbesondere subkutanen, intradermalen, intramuskulären oder intraperitonealen Abgabe eines Fluids gebildet werden.

Das Blockieren des zweiten Läufers, und damit das Halten der Verweilkanüle in der Verweilposition, kann lediglich durch Einwirkung des Steuerelementes auf den zweiten Läufer, insbesondere durch Ausüben einer Druckkraft, erfolgen. Damit wird eine weitere konstruktive Vereinfachung der Injektionsvorrichtung erzielt.

Der Einsatz von zusätzlichen Mitteln zum Halten der Verweilkanüle, beispielsweise von Rastelementen, entfällt.

Durch die Bewegung des Steuerelementes über den Steuerbereich kann zusätzlich, insbesondere durch eine zusätzliche Kurvenfläche des Steuerelementes, ein Hebelelement von einer ersten Position in zumindest eine zweite Position bewegbar, und damit insbesondere um eine Drehachse drehbar, sein. Dadurch können durch das Steuerelement weitere Funktionen kontrolliert werden. Beispielsweise ist es denkbar, dass nach dem Setzen der Verweilkanüle eine Ventil- oder Fördervorrichtung aktiviert wird, wodurch eine abzugebende Flüssigkeit zur Injektionsvorrichtung geleitet werden kann. Dies erhöht die Zuverlässigkeit einer derartigen Vorrichtung und macht diese weniger anfällig auf Fehlmanipulationen eines Benutzers.

Die vorliegende Erfindung betrifft ein Dosiergerät zur subkutanen, intradermalen, intramuskulären oder intraperitonealen (bevorzugt subkutanen) Abgabe eines Fluids. Das besagte Dosiergerät umfasst eine Fördervorrichtung zur Förderung des Fluids aus dem Innenraum eines Behälters. Das Fluid ist dabei mittels der Fördervorrichtung vom Behälter zu einer Abgabeöffnung förderbar. Das Dosiergerät kann ein Gehäuse mit einer aussenseitigen Kontaktfläche umfassen, über welche das Gerät insbesondere an den Körper eines Patienten anbringbar ist. Dadurch ist das Dosiergerät zur Abgabe eines Fluids nach einem vordefinierten Schema und/oder über einen längeren Zeitraum, insbesondere bei einem mobilen Patienten, geeignet.

Die vorliegende Erfindung betrifft ein derartiges Dosiergerät, welches alternativ oder zusätzlich zur subkutanen Abgabe zur intradermalen, intramuskulären oder intraperitonealen Abgabe eines Fluids geeignet ist.

Ein derartiges Dosiergerät kann eine Injektionsvorrichtung wie oben beschrieben umfassen. So können die Einstechkanüle und die Verweilkanüle in einer Ausgangsposition im Wesentlichen innerhalb des Gehäuses angeordnet und zum Setzen der Verweilkanüle durch eine Setzöffnung an der Kontaktfläche aus dem Gehäuse ausfahrbar sein. Dadurch kann insbesondere bei einem an den Körper eines Patienten angebrachten Dosiergerät das Setzen der Verweilkanüle vollautomatisch erfolgen. Dies erleichtert die Handhabung des Gerätes durch einen Patienten.

Wenn die Läufer der Injektionsvorrichtung über eine Führungsvorrichtung, vorzugsweise über eine gemeinsame Führungsvorrichtung, insbesondere über eine Linearführung, verschiebbar gelagert sind, kann die Führungsvorrichtung integraler Bestandteil des Gehäuses sein. Dadurch kann das Dosiergerät einfacher gefertigt werden.

Die Fördervorrichtung ist als ventillose Doppelkolbenpumpe ausgestaltet. Gerade der Einsatz einer Kolbenpumpe ist in diesem Zusammenhang vorteilhaft, da mit einer solchen höhere Drücke erzielt werden können als mit beispielsweise einer Peristaltikpumpe. Durch eine ventillose Ausführung lässt sich eine Kontamination des geförderten Fluids leichter verhindern. Eine Doppelkolbenpumpe ist besonders gut zur ventillosen Ausführung geeignet. Darüber hinaus gewährt dieser Pumpentyp eine grosse Vielseitigkeit in Bezug auf die realisierbaren Anwendungen. Ferner können gerade bei pharmazeutischen Formulierungen, die auf einem Biopolymer als Wirkstoff basieren, durch die Fördervorrichtung hervorgerufene Scherkräfte eine Zersetzung des Wirkstoffs hervorrufen. Dieser nachteilige Effekt kann durch die Verwendung der besagten Pumpen weitestgehend vermieden werden.

Die Fördervorrichtung umfasst einen Zylinder mit wenigstens einer Ansaugöffnung und wenigstens einer Auslassöffnung an einer Zylinderinnenwand sowie einen ersten und einen zweiten Kolben. Der erste Kolben und der zweite Kolben sind innerhalb des Zylinders in Längsrichtung verschiebbar gelagert. Dabei begrenzen der erste Kolben und der zweite Kolben zwischen ihren Stirnseiten gemeinsam mit einem Abschnitt der Zylinderinnenwand ein variables Fluidvolumen. Insbesondere parallel neben dem Zylinder ist eine Kurventrommel mit einer ersten und einer zweiten Kurvenstruktur angeordnet. Die erste Kurvenstruktur steht mit dem ersten Kolben und die zweite Kurvenstruktur mit dem zweiten Kolben in Wirkverbindung . Bei einer Rotation der Kurventrommel gibt die erste Kurvenstruktur die Hubbewegung des ersten Kolbens und die zweite Kurvenstruktur die Hubbewegung des zweiten Kolbens vor. Der Einsatz einer Doppelkolbenpumpe ermöglicht eine besonders einfache und wirkungsvolle Steuerung der beiden Kolben. Insbesondere wenn die Kurventrommel parallel neben dem Zylinder der Doppelkolbenpumpe angeordnet ist, kann eine besonders platzsparende und kompakte Ausführung erzielt werden.

Die Kurvenstrukturen können als Nuten oder als Wülste ausgebildet sein. Damit lässt sich eine besonders wirkungsvolle Kraftübertragung von der Kurventrommel auf die Kolben der Doppelkolbenpumpe erzielen. Die Kurventrommel kann mittels eines Drehantriebs antreibbar sein. Der Drehantrieb kann ein, insbesondere innerhalb der Kurventrommel angeordnetes, Planetengetriebe umfassen. Dadurch, dass das Planetengetriebe innerhalb der Kurventrommel angeordnet ist, kann die Kompaktheit der Vorrichtung weiter erhöht werden.

Die Ansaugöffnung und die Auslassöffnung können in Längsrichtung versetzt am Zylinder angeordnet sein. Dies stellt eine besonders vorteilhafte Ausführung einer Doppelkolbenpumpe dar. Die Ansaugöffnung kann mit dem Innenraum des Behälters in Fluidverbindung bringbar sein. Darüber hinaus kann die Auslassöffnung mit der Abgabeöffnung, insbesondere mit dem proximalen Endbereich der Einstechkanüle, in Fluidverbindung bringbar sein.

Ein derartiges Dosiergerät kann einen Behälter umfassen, wobei der Behälter vorzugsweise als kollabierbarer Beutel, Spritze oder Karpulle ausgebildet ist. Diese Behältnisse haben den Vorteil, dass aus ihnen unabhängig von ihrer Ausrichtung gegenüber der Schwerkraft eine Flüssigkeit förderbar ist, ohne dass es zu einem Ansaugen von Luft durch die Fördervorrichtung kommt. Dies ist insbesondere, wenn das Dosiergerät am Körper eines Patienten angebracht ist, vorteilhaft.

Die Fördervorrichtung kann zumindest eine weitere Öffnung, insbesondere eine Ansaug- und/oder Auslassöffnung, umfassen, die in Längsrichtung versetzt am Zylinder angeordnet ist. Damit können mit der Fördervorrichtung eine Vielzahl von weiteren Funktionen realisiert werden. Zum Beispiel ist es möglich, einen oder mehrere weitere Behälter an die Fördervorrichtung anzuschliessen, um die Rekonstitution eines Lyophilisates zu erzielen. Allerdings wäre es auch denkbar, dass an der Fördervorrichtung beispielsweise eine Analytikvorrichtung angeschlossen ist, mit der vor der Verabreichung eines Präparates an einen Patienten erst eine Analyse einer durch die Verweilkanüle angesaugten Körperflüssigkeit durchgeführt wird. Allerdings wäre auch die Analyse eines zu verabreichenden Fluids denkbar.

Das Dosiergerät kann ein Antriebsmodul und ein Abgabemodul umfassen, welche durch einen Benutzer verbindbar und/oder voneinander trennbar ausgebildet sind. Das Antriebsmodul kann zumindest Teile des Drehantriebs und/oder gegebenenfalls einen Setzantrieb der Injektionsvorrichtung umfassen. Das Abgabemodul kann zumindest den Behälter sowie die Fördervorrichtung und gegebenenfalls die Injektionsvorrichtung umfassen. Das Antriebsmodul kann darüber hinaus auch eine Batterie zur Versorgung der Antriebe sowie eine Steuerungseinheit zur Steuerung der Vorrichtung, insbesondere der Antriebe, umfassen. Ebenso können Kommunikationsmittel im Antriebsmodul vorgesehen sein, über welche eine externe Bedienungseinheit an die Steuereinheit anbindbar ist.

Das Antriebsmodul und das Abgabemodul können derart ausgebildet sein, dass die Antriebe über entsprechende Kopplungsmittel auf einfache Weise direkt oder indirekt an die Fördervorrichtung und/oder die Injektionsvorrichtung ankoppelbar sind. Hierzu können zum Beispiel form- und/oder kraftschlüssige Steckkupplungen zum Einsatz kommen. Die beiden Module können zum einfachen Austausch beispielsweise über eine Schnappkopplung aneinander ankoppelbar und/oder wieder trennbar sein.

Dies hat den Vorteil, dass das Antriebsmodul, welches keine hygienisch relevanten Komponenten enthält, vor seiner ersten Verwendung weder sterilisiert noch in einem Reinraum montiert werden muss. Damit können die Herstellungs- bzw. Anschaffungskosten der Vorrichtung reduziert werden. Zudem ist das Antriebsmodul problemlos wiederverwendbar, was auch die Betriebskosten des Dosiergerätes senkt. Auch gewährt diese Ausgestaltung mehr Flexibilität bei konstruktiven Modifikationen. Das Abgabemodul, welches sämtliche hygienisch relevanten Komponenten enthält, muss zwar sterilisiert werden. Es kann jedoch, da es keine überaus komplexen Komponenten enthält, unter geringerem Kostenaufwand hergestellt und nach einmaligem Gebrauch entsorgt werden. Da das Antriebsmodul und das Abgabemodul verbindbar und/oder voneinander trennbar ausgebildet sind, bleibt die Verwendung der Abgabevorrichtung durch den Benutzer dennoch denkbar einfach. Als Resultat hiervon können bei gleichbleibender Bedienerfreundlichkeit und Patientensicherheit die Kosten von Anschaffung und Betrieb des Dosiergerätes gesenkt werden.

Nicht Teil der Erfindung ist die Verwendung eines oben beschriebenen Dosiergerätes zur insbesondere subkutanen Abgabe eines Fluids.

Weitere Vorteile und Einzelmerkmale der Erfindung ergeben sich aus der nachstehenden Beschreibung eines Ausführungsbeispiels und aus den Zeichnungen.

Es zeigen schematisch:
- Figur 1:: Perspektivische Darstellung eines erfindungsgemässen Dosiergerätes;
- Figur 2:: Darstellung gemäss Figur 1, jedoch mit Schnitt durch das erfindungsgemässe Dosiergerät;
- Figur 3:: Darstellung gemäss den Figuren 1 und 2, jedoch mit anderem Schnitt durch das erfindungsgemässe Dosiergerät;
- Figur 4:: Darstellung gemäss den Figuren 1 bis 3, jedoch in Teilvergrösserung und mit weiterem Schnitt durch das erfindungsgemässe Dosiergerät;
- Figuren 5-18:: Sequenz von Schritten, welche das Setzen einer Verweilkanüle einer offenbarten Injektionsvorrichtung zeigt, wobei die Figuren mit ungerader Nummer das Dosiergerät jeweils in Draufsicht und die darauf folgenden Figuren mit geraden Nummern jeweils die Injektionsvorrichtung im entsprechenden Zustand in räumlicher Darstellung zeigen;
- Figur 19:: Perspektivische Schnittansicht eines erfindungsgemässen Dosiergerätes mit einer Verweilkanüle in Verweilposition;
- Figur 20:: Perspektivische Darstellung eines erfindungsgemässen Dosiergerätes mit Injektionsvorrichtung und Fördervorrichtung;
- Figur 21:: Perspektivische Darstellung der Fördervorrichtung eines erfindungsgemässen Dosiergerätes;
- Figur 22:: Schnitt durch eine Darstellung gemäss Figur 21;
- Figur 23:: Draufsicht auf einen Teilbereich eines erfindungsgemässen Dosiergerätes in einer ersten Betriebsposition;
- Figur 24:: Darstellung gemäss Figur 23, jedoch mit dem Dosiergerät in einer zweiten Betriebsposition;
- Figuren 25-28:: Perspektivische Darstellung einer Sequenz von Schritten, welche das Setzen einer Verweilkanüle eines alternativen Ausführungsbeispiels einer offenbarten Injektionsvorrichtung zeigt.

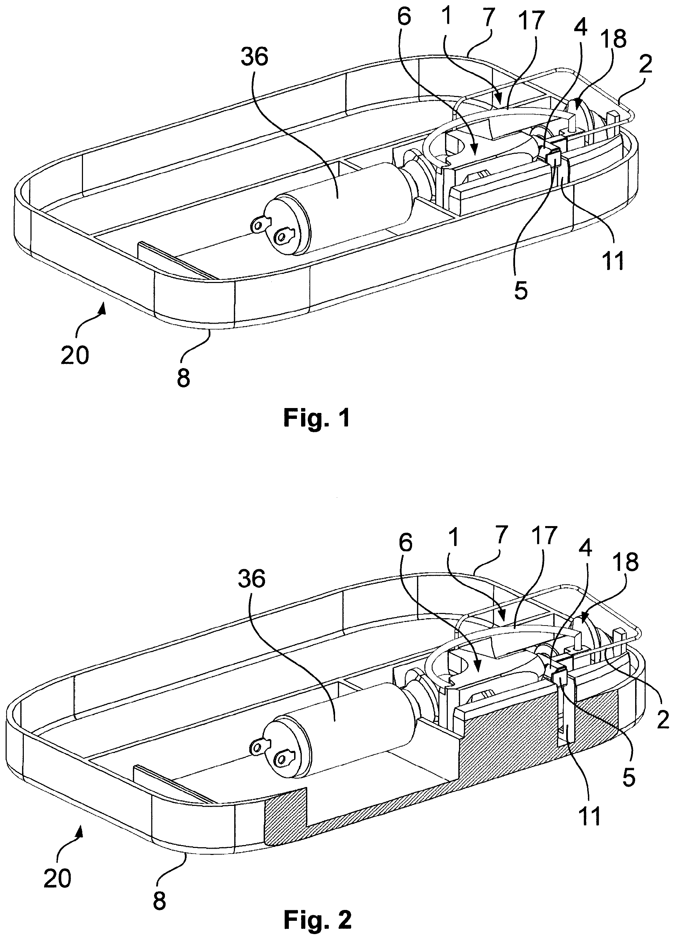

In Figur 1 ist ein erfindungsgemässes Dosiergerät 20 dargestellt, wobei zur besseren Übersicht lediglich die optionale Injektionsvorrichtung 1 mit der Kurventrommel 18 und dem Drehantrieb 36 dargestellt sind. Die Fördervorrichtung 21 sowie weitere Komponenten des Dosiergerätes 20, wie beispielsweise eine Steuerungseinheit oder eine Batterie, sind zur besseren Übersicht weggelassen. Das Dosiergerät 20 weist ein Gehäuse 7 auf, dessen Unterseite eine Kontaktfläche 8 zum Anbringen des Gerätes 20 an einem Patienten bildet. Die Injektionsvorrichtung 1 umfasst das Steuerungselement 6, welches mit dem ersten Läufer 4 und dem zweiten Läufer 5 in Wirkverbindung bringbar ist. Das Steuerungselement 6 ist über das Federelement 17 vorgespannt. Es wird allerdings durch die Kurventrommel 18 entgegen der Vorspannung zurückgehalten.

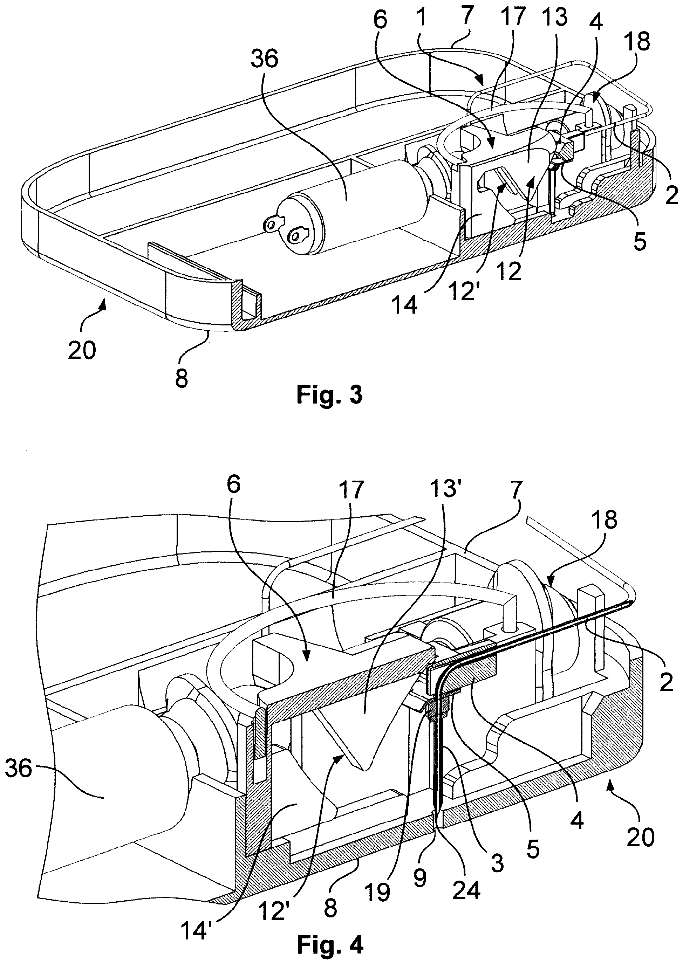

In der Schnittansicht gemäss Figur 2 ist die Linearführung 11 ersichtlich, mit welcher der erste Läufer 4 und der zweite Läufer 5 geführt sind. In Figur 3 ist das Steuerungselement 6 zu sehen. Es ist erkennbar, dass dieses als Paar von deckungsgleichen, verschiebbaren Kurventrägern 12, 12' ausgebildet ist. Vom vorderen Kurventräger 12 sind beide Abschnitte 13 und 14 sichtbar.

In Figur 4 ist der Teilbereich des Dosiergerätes mit der Injektionsvorrichtung 1 vergrössert dargestellt. Die Schnittebene ist im Vergleich zu den Figuren 2 und 3 weiter nach hinten versetzt, so dass diese nun in Längsrichtung durch die Einstechkanüle 2 und die Verweilkanüle 3 verläuft. Da der Schnitt auch durch das Steuerungselement 6 verläuft, ist nun der hintere Kurventräger 12' mit den beiden Abschnitten 13' und 14' sichtbar. Es ist zu erkennen, dass die Einstechkanüle 2 in der Ausgangsposition der Injektionsvorrichtung 1 in Längsrichtung innerhalb der Verweilkanüle 3 verläuft. An der Unterseite des Gehäuses 7 ist eine Setzöffnung 9 angebracht, welche die Kontaktfläche 8 durchdringt. Der zweite Läufer 5 ist als Halteblech ausgeführt, an welchem eine Dichtung 19 angebracht ist, die den Übergang zwischen der Setzkanüle 2 und der Verweilkanüle 3 abdichtet.

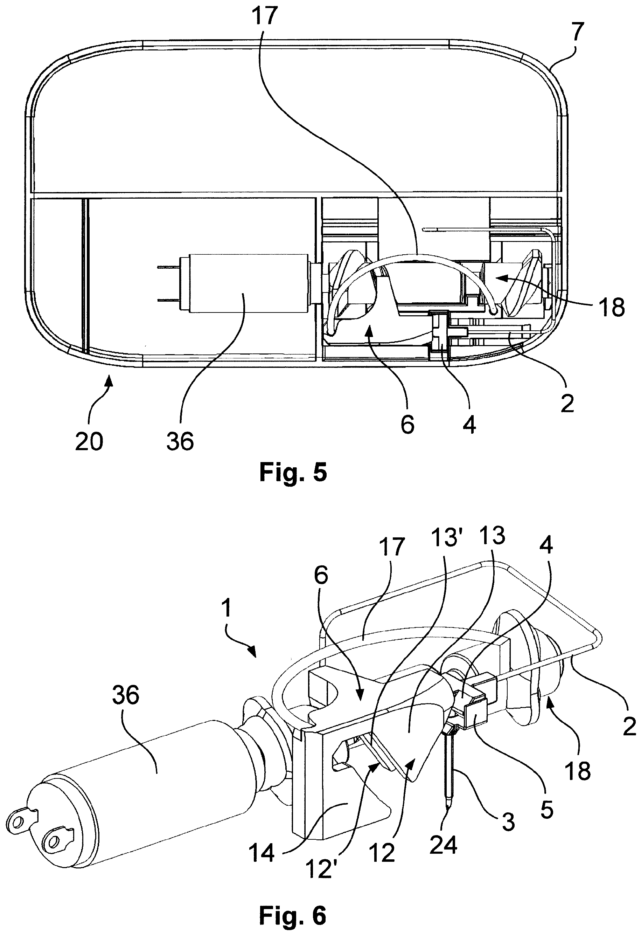

Die Figuren 5 und 6 zeigen die Injektionsvorrichtung 1 in ihrer Ausgangsposition. Die Kurventrommel 18 hat noch keine Drehbewegung vollführt. In Figur 6 ist zu erkennen, dass der erste Abschnitt 13 des Kurventrägers 12 bzw. der erste Abschnitt 13' des zweiten Kurventrägers 12' noch nicht mit dem ersten Läufer 4 und dem zweiten Läufer 5 in Wirkverbindung stehen.

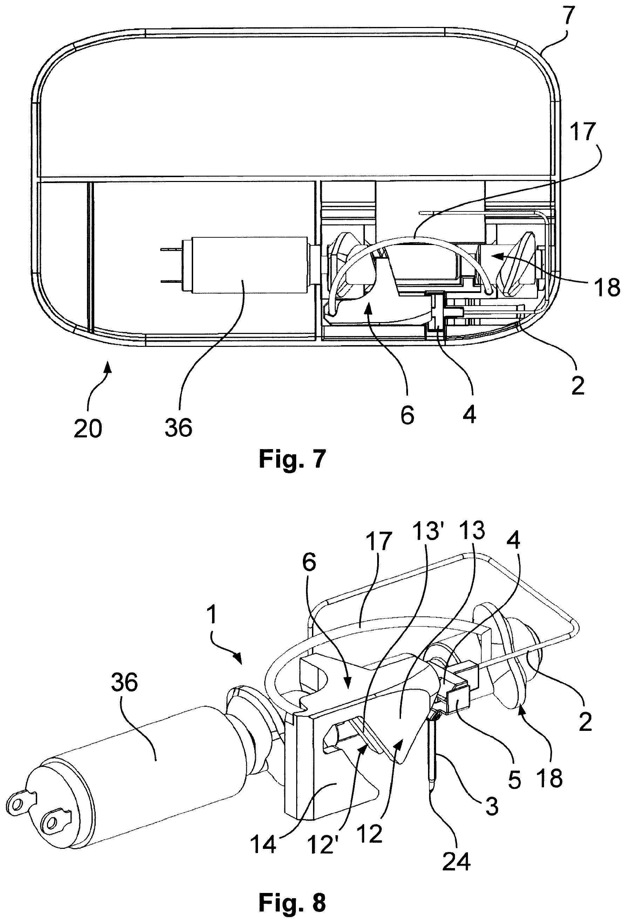

Die Figuren 7 und 8 zeigen die Injektionsvorrichtung 1 zu Beginn des Setzvorgangs. Die Kurventrommel 18 hat sich bereits um einige Grade gedreht. Die ersten Bereiche 13, 13' der Kurventräger 12, 12' stehen nun mit dem zweiten Läufer 5 in Wirkverbindung.

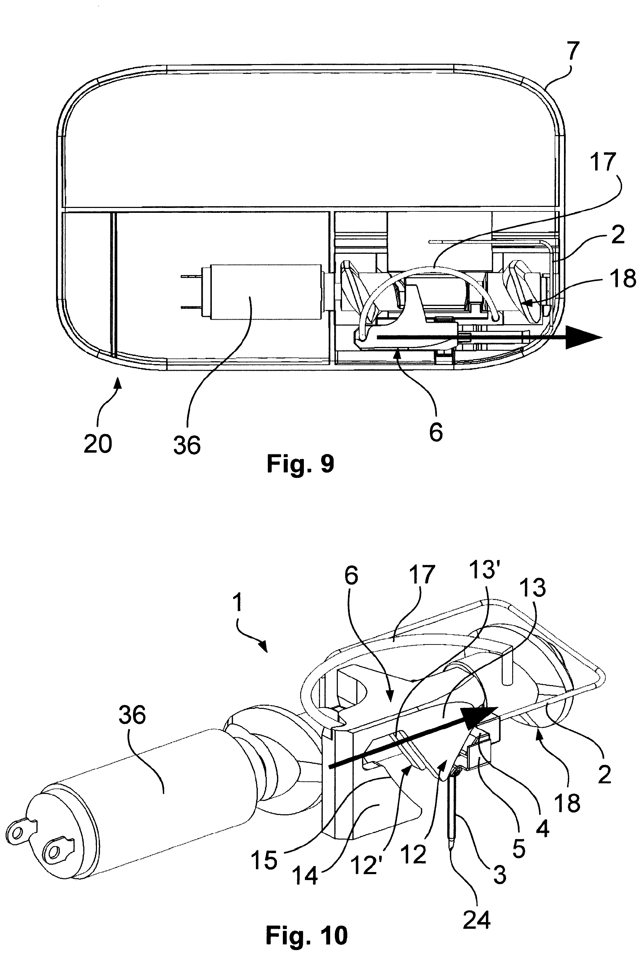

Die Figuren 9 und 10 zeigen die Injektionsvorrichtung 1 während des eigentlichen Setzvorgangs. In Figur 9 ist zu erkennen, dass sich die Kurventrommel 18 nun soweit gedreht hat, dass sie das Steuerungselement 6 nicht mehr entgegen der Vorspannung des Federelementes 17 zurückhält. Entsprechend kann sich das Steuerelement 6 frei bewegen, wodurch die ersten Abschnitte 13, 13' der Kurventräger 12, 12' den ersten Läufer 4 sowie den zweiten Läufer 5 nach unten drücken.

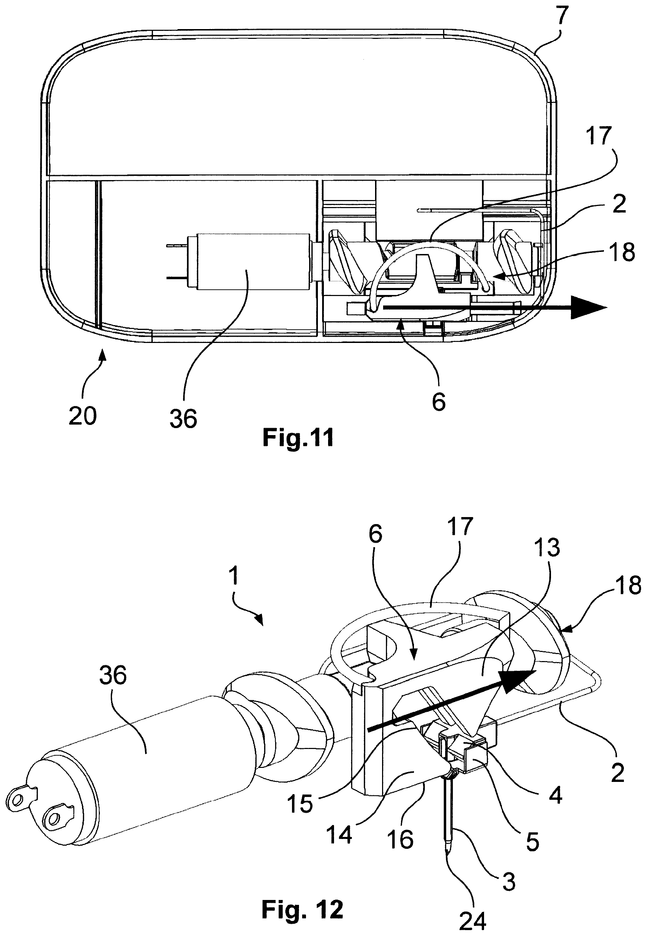

In den Figuren 11 und 12 sind der erste Läufer 4 und der zweite Läufer 5 an ihrem unteren Anschlagspunkt angekommen. Die Einstechkanüle 2 ist damit gesetzt. In Figur 11 ist zu erkennen, dass das Steuerelement 6 erst etwa die Hälfte seines Weges zurückgelegt hat und sich durch die Federvorspannung weiterverschiebt.

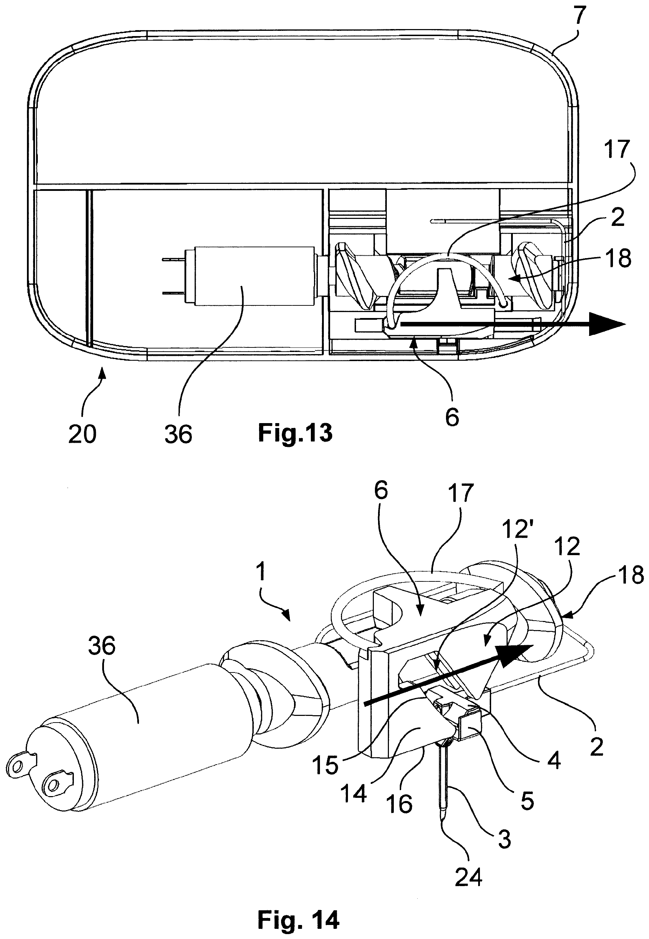

In den Figuren 13 und 14 stehen der erste Läufer 4 und der zweite Läufer 5 nun mit den zweiten Abschnitten 14, 14' der Kurventräger 12, 12' in Wirkverbindung. Dabei wirkt die Oberseite 15 bzw. 15' der zweiten Abschnitte 14, 14' auf den ersten Läufer 4, wodurch die Setzkanüle 2 aus der Verweilkanüle 3 nach oben zurückgezogen wird. Auf den zweiten Läufer 5 wirkt die Unterseite 16 bzw. 16' der zweiten Abschnitte 14, 14'. Dies führt zu einem Arretieren des zweiten Läufers 5, und damit der Verweilkanüle 3, in ihrer Verweilposition.

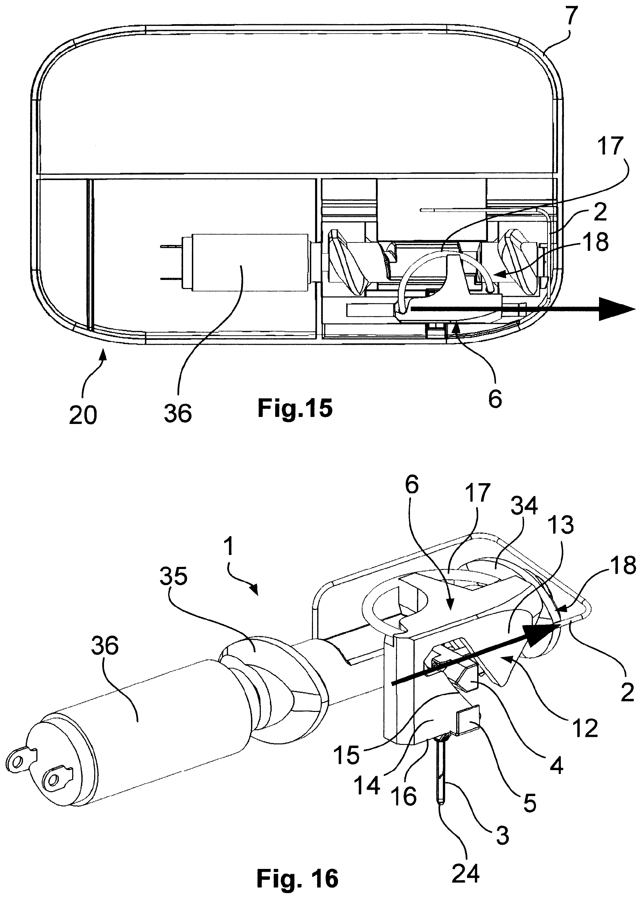

Die Figuren 15 und 16 zeigen das Zurückziehen nach oben der Setzkanüle 2 aus der Verweilkanüle 3. Auch hier wird der erste Läufer 4 von der Unterseite 15 bzw. 15' der zweiten Abschnitte 14, 14' nach oben gedrückt.

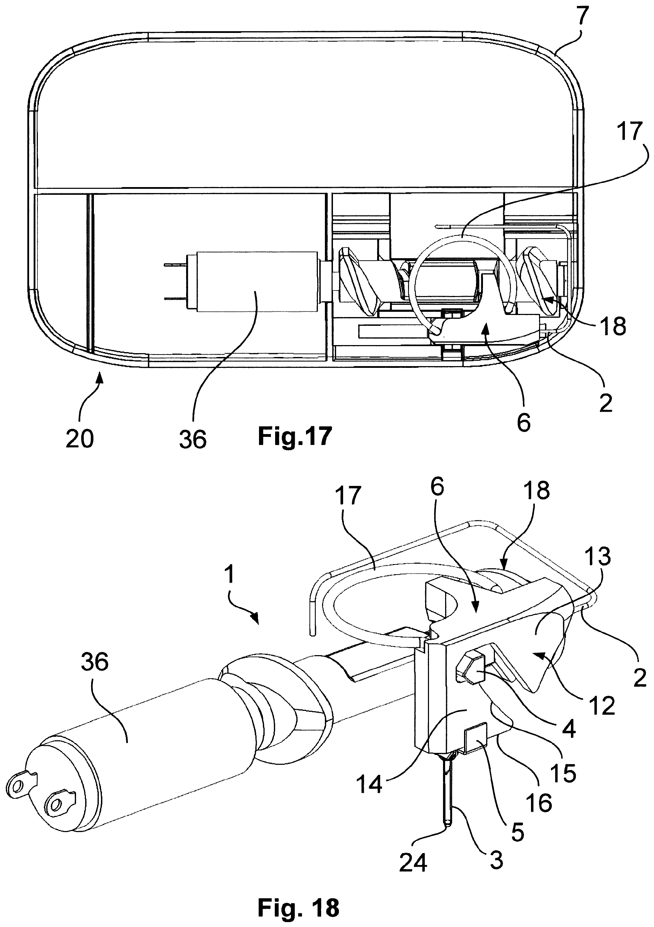

In den Figuren 17 und 18 hat das Steuerungselement 16 seinen Anschlag an der Kurventrommel 18 erreicht. Es ist nicht mehr weiter durch das Federelement 17 bewegbar. Es ist zu erkennen, dass der erste Läufer 4 seine Endposition erreicht hat, während dem der zweite Läufer 5, und damit die Verweilkanüle 3, noch immer in der Verweilposition arretiert sind.

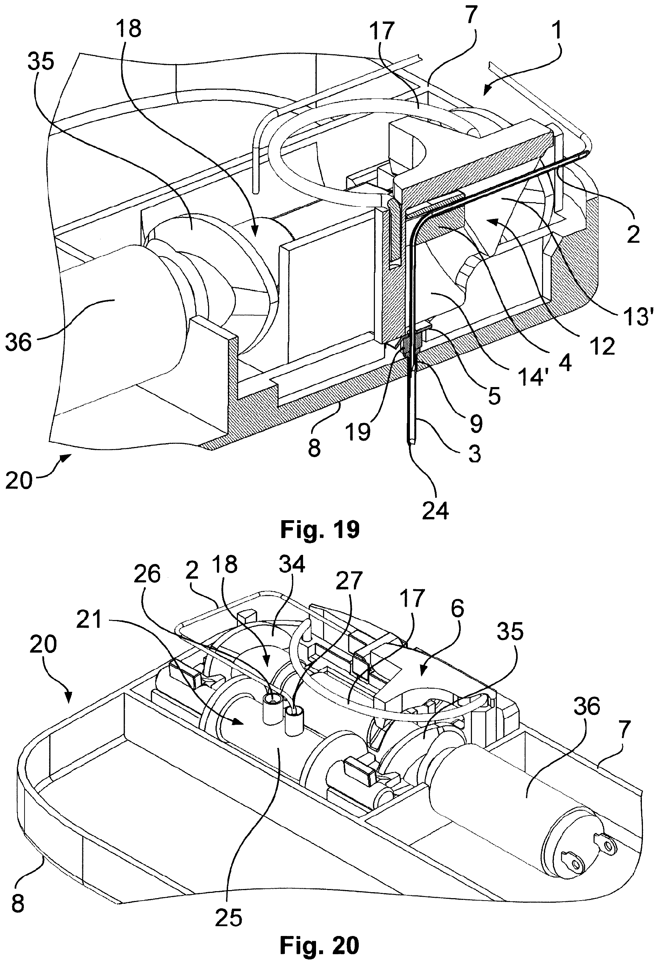

Die Figur 19 zeigt eine Schnittansicht der Injektionsvorrichtung 1 im Dosiergerät 20 in ihrer Endposition. Die Verweilkanüle 3 ragt nun aus der Setzöffnung 9 hinaus, während die Einstechkanüle 2 im Wesentlichen zurückgezogen ist. Zudem ist das Dichtungselement 19 erkennbar, welches durch den zweiten Läufer 5 und das Gehäuse 7 des Gerätes 20 gequetscht wird. Dadurch kann eine bessere Abdichtung beim Übergang von der Setzkanüle 2 zur Verweilkanüle 3 erzielt werden.

Figur 20 zeigt eine räumliche Darstellung des erfindungsgemässen Dosiergerätes 20, nun mit der Fördervorrichtung 21. Es ist zu erkennen, dass die Kurventrommel 18 sowohl mit der optionalen Injektionsvorrichtung 1 als auch mit der Fördervorrichtung 21 in Wirkverbindung steht. Die Kurventrommel 18 dient damit gleichzeitig der Steuerung der Injektionsvorrichtung 1 und dem Antrieb der Fördervorrichtung 21. Der proximale Endbereich der Einstechkanüle 21 ist fluidmässig mit der Ausgangsöffnung 27 der Fördervorrichtung 21 verbunden.

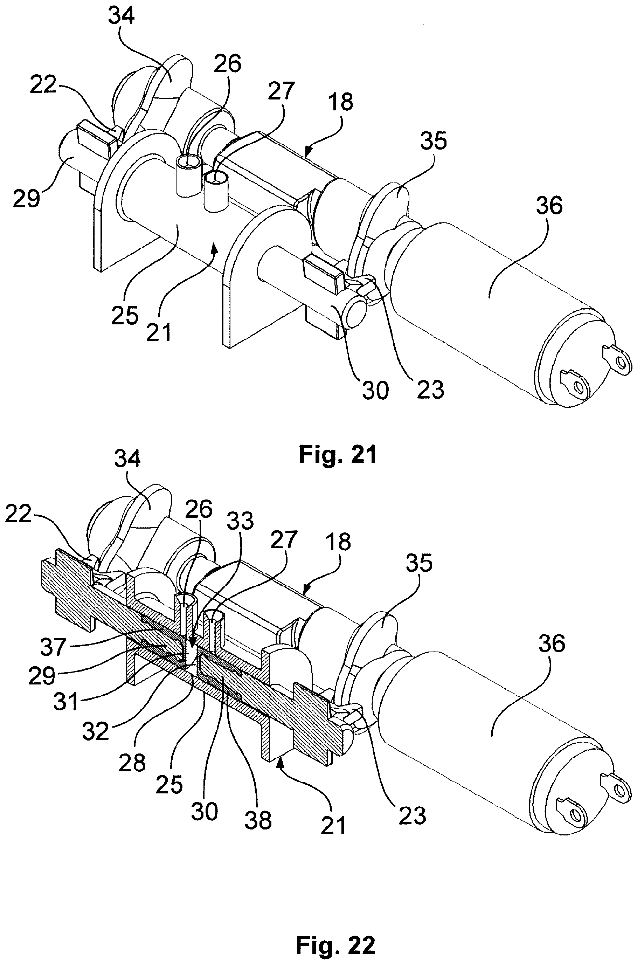

In Figur 21 ist die Fördervorrichtung 21 in Kombination mit der Kurventrommel 18 und dem Drehantrieb 36 in Alleinstellung dargestellt. Es ist zu erkennen, dass die Fördervorrichtung 21 als Doppelkolbenpumpe mit den Zylindern 25 sowie den Kolben 29 und 30 ausgeführt ist. An den Kolben 29 und 30 sind jeweils gabelförmige Elemente 22 und 23 angebracht, in welche wulstartige Kurvenelemente 34 und 35 der Kurventrommel 18 eingreifen.

Figur 22 zeigt eine der Figur 21 entsprechende Abbildung mit einem Schnitt entlang der Längsmittelachse des Zylinders 25. Es ist zu erkennen, dass die Kolben 29 und 30 in ihren Endbereichen mit den Dichtungselementen 37 und 38 ausgestattet sind. Die Stirnseiten 31 und 32 der Kolben 29 und 30 bilden gemeinsam mit der Innenwand 28 des Zylinders 25 ein variables Fluidvolumen 33. Die Eingangsöffnung 26 und die Auslassöffnung 27 der Fördervorrichtung 21 sind in Längsrichtung versetzt am Zylinder 25 angebracht.

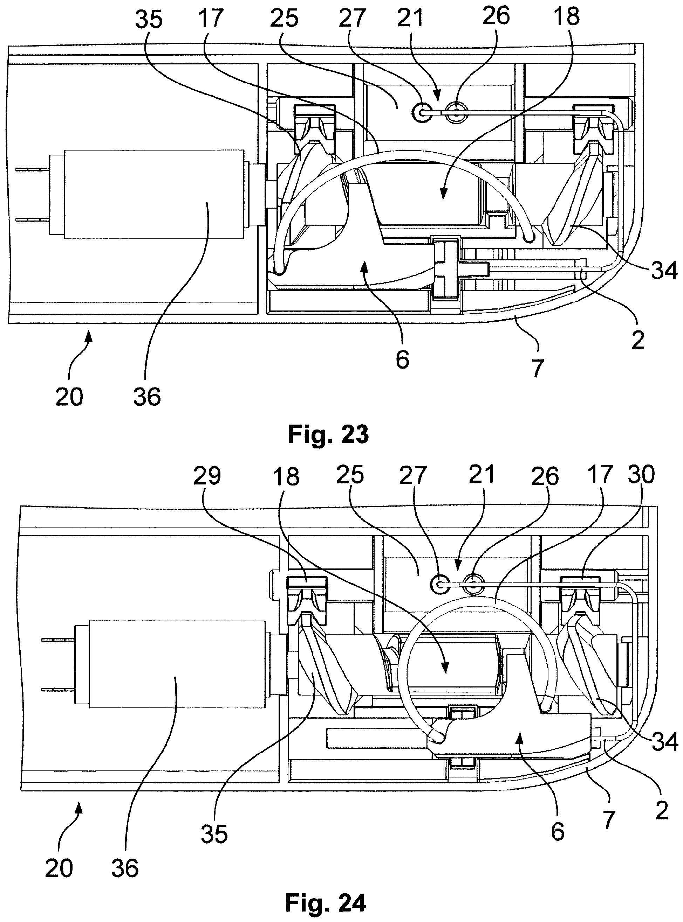

Die Figuren 23 und 24 zeigen eine Draufsicht auf einen Teilbereich des erfindungsgemässen Dosiergerätes vor und nach dem Setzvorgang. Es ist zu erkennen, dass der Setzvorgang mit dem Drehantrieb 36 über eine Rotation der Kurventrommel 18 ausgelöst wird. Das Verschieben des Steuerelements 6 erfolgt allerdings hauptsächlich durch die Vorspannung des Federelementes 17. Durch die Rotation der Kurventrommel 18 kommt es zudem zu einer Längsverschiebung der Zylinder 29 und 30 innerhalb des Kolbens 25.

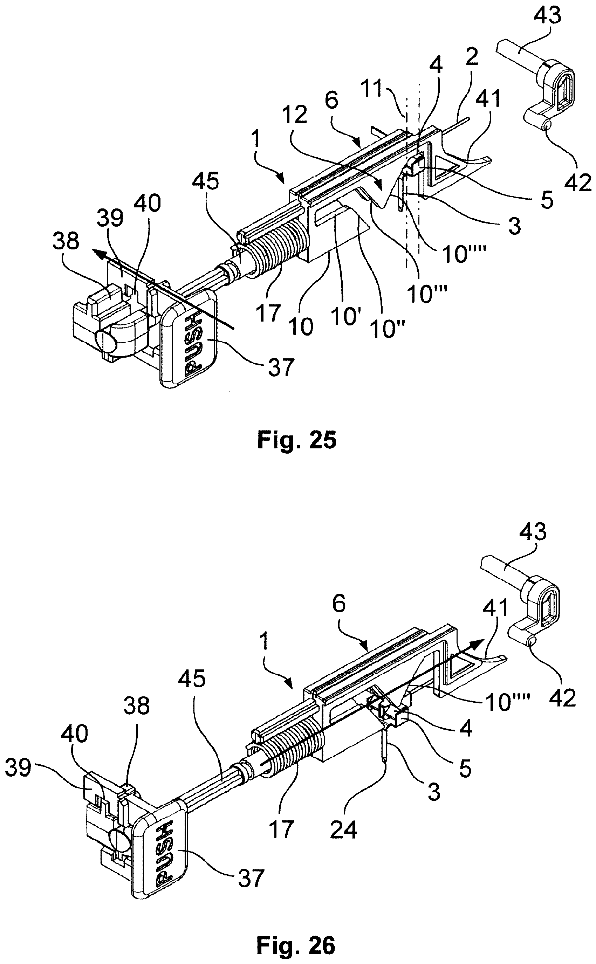

Die Figuren 25 bis 28 zeigen ein alternatives Ausführungsbeispiel der optionalen Injektionsvorrichtung 1. In Figur 25 ist die besagte Vorrichtung 1 in ihrer Ausgangsposition ersichtlich. Das Steuerelement 6 ist über ein Federelement 17, das hier als Schraubenfeder ausgebildet ist, vorgespannt. Das Steuerelement 6 wird durch ein Anschlagelement 38, welches auf einer Anschlagplatte 39 aufliegt, gegen die Vorspannung zurückgehalten. Durch Betätigen der Auslösetaste 37 (in Pfeilrichtung) wird die Anschlagplatte 39 in einer Richtung senkrecht zur Verschiebungsrichtung des Steuerelementes 6 verschoben. Dadurch kann das Anschlagelement 38 durch die Aussparung 40 in der Anschlagplatte 39 gleiten und den eigentlichen Setzvorgang auslösen.

In der Figur 25 sind zusätzlich die Kurvenflächen 10, 10', 10", 10‴, 10"" des Kurventrägers 12 eingezeichnet, welche die Bewegung des ersten Läufers 4 und des zweiten Läufers 5 über den Steuerbereich vorgeben.

In den Figuren 25 bis 28 sind lediglich Teile der Injektionsvorrichtung 1 dargestellt. Die nicht gezeigten Teile entsprechen in der Regel denjenigen gemäss den Figuren 1 bis 25. Insbesondere sind die Linearführungen 11 (in Figur 25 lediglich strichliert angedeutet) im Wesentlichen identisch zu denjenigen in den vorhergehenden Figuren.

In Figur 26 ist das Steuerelement 6 bereits über den ersten Teil das Steuerbereiches verschoben (in Pfeilrichtung). Dies hat mittels der Fläche 10"" zu einem simultan-gleichgerichteten Verschieben der beiden Läufer 4, 5 und damit zu einem Setzen der Verweilkanüle 3 mithilfe der Einstechkanüle 2 geführt.

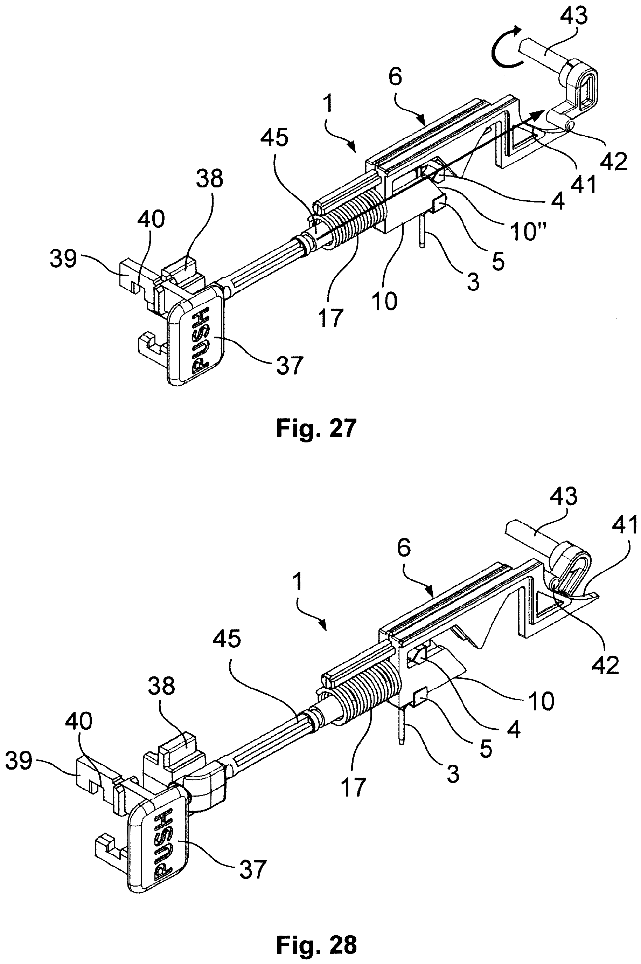

Es versteht sich von selbst dass es während des Verschiebens des Steuerelementes 6 zu einer Expansion der Schraubenfeder 17 kommt. Aus zeichnungstechnischen Gründen ist diese in den Figuren 25 bis 28 allerdings immer im komprimierten Zustand gezeigt. Die Figur 27 zeigt das Verschieben des Steuerelementes 6 über einen zweiten Teil des Steuerbereiches. Dabei wird zum einen ein Blockieren des als Halteblech ausgeführten zweiten Läufers 5, und damit ein Halten der Verweilkanüle 3 in der Verweilposition, lediglich durch Einwirkung, namentlich durch Herabdrücken, durch das Steuerelement 6 mit der Kurvenfläche 10 erzielt. Zum anderen wird mittels der Kurvenfläche 10" ein Zurückziehen der Einstechkanüle 2 aus dem distalen Endbereich der Verweilkanüle 3 erzielt.

Das Steuerelement 6 weist eine zusätzliche Kurvenfläche 41 auf, die im zweiten Teil des Steuerbereiches auf das Hebelelement 42 wirkt. Damit ist das Hebelelement 42 von der in Figur 27 gezeigten ersten Position in eine in Figur 28 abgebildete zweite Position bewegbar, wodurch es zu einer Rotation einer Drehachse 43 kommt. Das Steuerelement 6 kann damit weitere Funktionen einer entsprechenden Vorrichtung, beispielsweise eines Dosiergerätes kontrollieren. So kann eine Aktivierung einer Ventil- oder Fördervorrichtung, um ein Fluid zur Injektionsvorrichtung zu leiten, realisiert werden.

## Patentansprüche

1. Dosiergerät (20) zur subkutanen, intradermalen, intramuskularen oder intraperitonealen Abgabe eines Fluids,
umfassend eine Fördervorrichtung (21) zur Förderung des Fluids aus dem Innenraum eines Behälters, wobei das Fluid mittels der Fördervorrichtung (21) vom Behälter zu einer Abgabeöffnung (24) förderbar ist; wobei die Fördervorrichtung (21) als ventillose Doppelkolbenpumpe ausgestaltet ist; wobei die Fördervorrichtung (21) einen Zylinder (25) mit wenigstens einer Ansaugöffnung (26) und wenigstens einer Auslassöffnung (27) an einer Zylinderinnenwand (28), sowie einen ersten Kolben (29) und einen zweiten Kolben (30) umfasst, wobei die Ansaugöffnung (26) und die Auslassöffnung (27) in Längsrichtung versetzt am Zylinder (25) angeordnet sind, wobei der erste Kolben (29) und der zweite Kolben (30) innerhalb des Zylinders (25) in Längsrichtung verschiebbar gelagert sind, wobei der erste Kolben (29) und der zweite Kolben (30) zwischen ihren Stirnseiten (31,32) gemeinsam mit einem Abschnitt der Zylinderinnenwand (28) ein variables Fluidvolumen (33) begrenzen,
**dadurch gekennzeichnet, dass** insbesondere parallel neben dem Zylinder (25) eine Kurventrommel (18) mit einer ersten und einer zweiten Kurvenstruktur (34, 35) angeordnet ist,wobei die erste Kurvenstruktur (34) mit dem ersten Kolben (29) und die zweite Kurvenstruktur (35) mit dem zweiten Kolben (30) in Wirkverbindung steht wobei an den Kolben (29) und (30) jeweils gabelförmige Elemente (22) und (23) angebracht sind, in welche jeweils die Kurvenstruktur (34) und (35) der Kurventrommel (18) eingreifen und bei einer Rotation der Kurventrommel (18) die erste Kurvenstruktur (34) die Hubbewegung des ersten Kolbens (29) und die zweite Kurvenstruktur (35) die Hubbewegung des zweiten Kolbens (30) vorgibt.

2. Dosiergerät (20) nach Anspruch 1, umfassend eine Injektionsvorrichtung (1) zur subkutanen Abgabe eines Fluids, umfassend eine Einstechkanüle (2) und eine Verweilkanüle (3), wobei in einer Ausgangsposition ein distaler Endbereichder Einstechkanüle (2) koaxial im Inneren der Verweilkanüle (3) verläuft, **dadurch gekennzeichnet, dass** die Vorrichtung (1) einen ersten und einen zweiten verschiebbar gelagerten Läufer (4, 5) umfasst, wobei der erste Läufer (4) mit der Einstechkanüle (2) und der zweite Läufer (5) mit der Verweilkanüle (3) verbunden ist, und dass die Injektionsvorrichtung (1) ferner ein über einen vordefinierten Steuerbereich bewegbares Steuerelement (6) umfasst, das zum Verschieben des ersten Läufers (4) und des zweiten Läufers (5) mit diesen in Wirkverbindung bringbar ist, wobei das Steuerelement (6) derart ausgebildet ist, dass es in einem ersten Teil des Steuerbereiches ein gleichgerichtetes, insbesondere simultanes, Verschieben der beiden Läufer (3, 4), und damit ein Setzen der Verweilkanüle (3) bewirkt und in einem zweiten Teil des Steuerbereiches ein Blockieren des zweiten Läufers (5), und damit ein Halten der Verweilkanüle (3) in einer Verweilposition, sowie ein Zürückverschieben des ersten Läufers (4), und damit ein Zürückziehen der Einstechkanüle (2) aus dem distalen Endbereich der Verweilkanüle (3) in eine Endposition, bewirkt, wobei die Läufer über eine Führungsvorrichtung, vorzugsweise über eine gemeinsame Führungsvorrichtung, verschiebbar gelagert sind.

3. Dosiergerät nach Anspruch 2, wobei die Läufer über eine Linearführung (11), verschiebbar gelagert sind.

4. Dosiergerät nach Anspruch 3, wobei die Läufer über eine gemeinsame Linearführung (11) verschiebbar gelagert sind und die Linearführung (11) in einer Setzrichtung parallel zu den distalen Endbereichen der Einstechkanüle (2) undder Verweilkanüle (3) ausgerichtet ist.

5. Dosiergerät nach einem der Ansprüche 3 oder 4, wobei das Steuerelement (6) in einer Richtung, insbesondere im Wesentlichen, senkrecht zu derjenigen der Linearführung(11) bewegbar, insbesondere verschiebbar, gelagert ist.

6. Dosiergerät nach einem der Anspruche 2 bis 5, wobei das Steuerelement (6) als verschiebbarer Kurventräger (12) ausgebildet ist.

7. Dosiergerät nach Anspruch 6, wobei das Steuerelement (6) einen ersten und einen zweiten Abschnitt (13, 14) umfasst, wobei der erste Abschnitt (13) ein gleichgerichtetes, insbesondere simultanes, Verschieben der beiden Läufer (4, 5), und damit ein Setzen der Verweilkanüle (3), bewirkt und der zweite Abschnitt ein Blockieren des zweiten Läufers (5), und damit ein Halten der Verweilkanüle (3) in einer Verweilposition, sowie ein Zürückverschieben des ersten Läufers (4), und damit ein Zürückziehenden Einstechkanüle (2) aus dem distalen Endbereich der Verweilkanüle (3) in eine Endposition, bewirkt, wobei insbesondere unterschiedliche Seiten (15, 16) des zweiten Abschnittes (14) auf den ersten und auf den zweiten Läufer (4, 5) wirken.

8. Dosiergerät (20) nach einem der Ansprüche 2 bis 7,wobei die Einstechkanüle (2) und die Verweilkanüle (3) in der Ausgangsposition im Wesentlichen innerhalb eines Gehäuses (7) angeordnet und zum Setzen der Verweilkanüle (3) durch eine Setzöffnung (9) in der Kontaktfläche (8) aus demGehäuse (7) ausfahrbar sind.

9. Dosiergerät (20) gemäss einem der vorhergehenden Ansprüche, wobei die Ansaugöffnung (26) mit dem Innenraum des Behälters in Fluidverbindung bringbar ist.

10. Dosiergerät (20) gemäss einem der vorhergehenden Ansprüche, wobei die Fördervorrichtung (5) zumindest eine weitere Öffnung, insbesondere eine Ansaug und/oder Auslassöffnung, umfasst, die in Längsrichtung versetzt am Zylinder angeordnet ist, umfassend mehrere Behälter wobei jede Ansaugöffnung mitdem Innenraum eines separaten, ihr zugeordneten Behälters in Fluidverbindung bringbar ist.

## Claims

1. A dosing apparatus (20) for dispensing a fluid subcutaneously, intradermally, intramuscularly or intraperitoneally,
comprising a delivery device (21) for delivering the fluid from the interior of a container, the fluid being deliverable by means of the delivery device (21) from the container to a dispensing opening (24), the delivery device (21) being designed as a valveless double piston pump, the delivery device (21) comprising a cylinder (25), including at least one intake opening (26) and at least one discharge opening (27) on an inner cylinder wall (28), as well as a first piston (29) and a second piston (30), the intake opening (26) and the discharge opening (27) being arranged at the cylinder (25) offset in the longitudinal direction, the first piston (29) and the second piston (30) being mounted inside the cylinder (25) displaceably in the longitudinal direction, the first piston (29) and the second piston (30) between the end faces (31, 32) thereof, together with a section of the inner cylinder wall (28), delimiting a variable fluid volume (33),
**characterized in that** a barrel cam (18), including a first and a second cam structure (34, 35), is arranged in particular parallel next to the cylinder (25), the first cam structure (34) being operatively connected to the first piston (29) and the second cam structure (35) being operatively connected to the second piston (30), fork-shaped elements (22) and (23) being in each case attached at the pistons (29) and (30), in each of which the cam structures (34) and (35) of the barrel cam (18) engage and, during a rotation of the barrel cam (18), the first cam structure (34) predefining the stroke movement of the first piston (29) and the second cam structure (35) predefining the stroke movement of the second piston (30).

2. The dosing apparatus (20) according to claim 1, comprising an injection device (1) for subcutaneously dispensing a fluid, comprising a piercing cannula (2) and an indwelling cannula (3), in an initial position a distal end region of the piercing cannula (2) extending coaxially inside the indwelling cannula (3), **characterized in that** the device (1) comprises a first and a second displaceably mounted runner (4, 5), the first runner (4) being connected to the piercing cannula (2) and the second runner (5) being connected to the indwelling cannula (3), and that the injection device (1) furthermore comprises a control element (6), which can be moved across a predefined control area and, for displacing the first runner (4) and the second runner (5), can be brought into operative connection therewith, the control element (6) being designed so as to effect, in a first portion of the control area, a unidirectional, in particular simultaneous, displacement of the two runners (3, 4), and thus an insertion of the indwelling cannula (3) and, in a second portion of the control area, a blocking of the second runner (5), and thus a retention of the indwelling cannula (3), in a dwelling position, as well as a back-displacement of the first runner (4), and thus a retraction of the piercing cannula (2) from the distal end region of the indwelling cannula (3) into an end position, the runners being displaceably mounted by way of a guide device, preferably by way of a shared guide device.

3. The dosing apparatus according to claim 2, wherein the runners are displaceably mounted by way of a linear guide (11).

4. The dosing apparatus according to claim 3, wherein the runners are displaceably mounted by way of a shared linear guide (11), and the linear guide (11) is aligned in an insertion direction parallel to the distal end regions of the piercing cannula (2) and of the indwelling cannula (3).

5. The dosing apparatus according to one of claims 3 or 4, wherein the control element (6) is mounted so as to be movable, in particular displaceable, in one direction, in particular substantially perpendicular to that of the linear guide (11).

6. The dosing apparatus according to one of claims 2 to 5, wherein the control element (6) is designed as a displaceable cam carrier (12).

7. The dosing apparatus according to claim 6, wherein the control element (6) comprises a first and a second section (13, 14), the first section (13) effectuating a unidirectional, in particular simultaneous, displacement of the two runners (4, 5), and thus an insertion of the indwelling cannula (3), and the second section effectuating a blocking of the second runner (5), and thus a retention of the indwelling cannula (3) in a dwelling position, as well as a back-displacement of the first runner (4), and thus a retraction of the piercing cannula (2) from the distal end region of the indwelling cannula (3) into an end position, in particular different sides (15, 16) of the second section (14) acting on the first runner and on the second runner (4, 5).

8. The dosing apparatus (20) according to one of claims 2 to 7, wherein, in the initial position, the piercing cannula (2) and the indwelling cannula (3) are arranged substantially within a housing (7) and, for the purpose of inserting the indwelling cannula (3), can be moved out of the housing (7) through an insertion opening (9) in the contact surface (8).

9. The dosing apparatus (20) according to one of the preceding claims, wherein the intake opening (26) can be brought into fluid connection with the interior of the container.

10. The dosing apparatus (20) according to one of the preceding claims, wherein the delivery device (5) includes at least one further opening, in particular an intake and/or discharge opening, which is arranged at the cylinder offset in the longitudinal direction, comprising a plurality of containers, it being possible to bring each intake opening into fluid connection with the interior of a separate container assigned thereto.

## Revendications

1. Appareil de dosage (20) pour l'administration sous-cutanée, intradermique, intramusculaire ou intrapéritonéale d'un fluide,
comprenant un dispositif de transport (21) pour le transport du fluide de l'espace intérieur d'un réservoir, le fluide pouvant être transporté au moyen du dispositif de transport (21) du réservoir à une ouverture d'évacuation (24) ; dans lequel le dispositif de transport (21) est conçu comme une pompe à double piston sans soupape ; le dispositif de transport (21) comprenant un cylindre (25) comprenant au moins une ouverture d'aspiration (26) et au moins une ouverture de sortie (27) sur une paroi interne de cylindre (28), ainsi qu'un premier piston (29) et un deuxième piston (30), l'ouverture d'aspiration (26) et l'ouverture de sortie (27) étant disposées décalées dans la direction longitudinale sur le cylindre (25), le premier piston (29) et le deuxième piston (30) étant logés de manière coulissante dans la direction longitudinale à l'intérieur du cylindre (25), le premier piston (29) et le deuxième piston (30) limitant, entre leurs faces avant (31, 32), ensemble avec une section de la paroi interne de cylindre (28), un volume de fluide (33) variable,
**caractérisé en ce qu'un** tambour à cames (18) comprenant une première et une deuxième structure à cames (34, 35) est disposé, en particulier en parallèle, à côté du cylindre (25), la première structure à cames (34) étant en liaison active avec le premier piston (29) et la deuxième structure à cames (35) étant en liaison active avec le deuxième piston (30), des éléments en forme de fourchette (22) et (23) étant montés respectivement sur les pistons (29) et (30), dans lesquels respectivement entrent en prise les structures à cames (34) et (35) du tambour à cames (18) et, lors d'une rotation du tambour à cames (18), la première structure à cames (34) prédéterminant la course du premier piston (29) et la deuxième structure à cames (35) prédéterminant la course du deuxième piston (30).

2. Appareil de dosage (20) selon la revendication 1, comprenant un dispositif d'injection (1) pour l'administration sous-cutanée d'un fluide, comprenant une canule insérable (2) et une canule permanente (3), dans une position initiale une zone terminale distale de la canule insérable (2) s'étendant de manière coaxiale à l'intérieur de la canule permanente (3), **caractérisé en ce que** le dispositif (1) comprend un premier et un deuxième coulisseaux (4, 5) logés de manière coulissante, le premier coulisseau (4) étant connecté à la canule insérable (2) et le deuxième coulisseau (5) étant connecté à la canule permanente (3), et **en ce que** le dispositif d'injection (1) comprend en outre un élément de commande (6) pouvant être déplacé à travers une zone de commande prédéfinie, lequel, pour le coulissement du premier coulisseau (4) et du deuxième coulisseau (5), peut être mis en liaison active avec ceux-ci, l'élément de commande (6) étant conçu de telle façon qu'il suscite, dans une première partie de la zone de commande, un coulissement parallèle, en particulier simultané, des deux coulisseaux (3, 4) et ainsi l'enfoncement de la canule permanente (3) et qu'il suscite, dans une deuxième partie de la zone de commande, le blocage du deuxième coulisseau (5), et ainsi un maintien de la canule permanente (3) dans une position permanente, ainsi qu'un coulissement en arrière du premier coulisseau (4) et ainsi un retrait de la canule insérable (2) hors de la zone terminale distale de la canule permanente (3) dans une position finale, les coulisseaux étant logés de manière coulissante sur un dispositif de guidage, de préférence sur un dispositif de guidage commun.

3. Appareil de dosage selon la revendication 2, les coulisseaux étant logés de manière coulissante sur un guidage linéaire (11).

4. Appareil de dosage selon la revendication 3, les coulisseaux étant logés de manière coulissante sur un guidage linéaire (11) commun et le guidage linéaire (11) étant orienté, dans une direction d'enfoncement, parallèle aux zones terminales distales de la canule insérable (2) et de la canule permanente (3).

5. Appareil de dosage selon l'une quelconque des revendications 3 ou 4, l'élément de commande (6) étant logé de manière mobile, en particulier coulissante, dans une direction, en particulier essentiellement, perpendiculaire à celle du guidage linéaire (11).

6. Appareil de dosage selon l'une quelconque des revendications 2 à 5, l'élément de commande (6) étant conçu comme un porte-came (12) coulissant.

7. Appareil de dosage selon la revendication 6, l'élément de commande (6) comprenant une première et une deuxième sections (13, 14), la première section (13) suscitant un coulissement parallèle, en particulier simultané, des deux coulisseaux (4, 5) et ainsi l'enfoncement de la canule permanente (3) et la deuxième section suscitant le blocage du deuxième coulisseau (5), et ainsi un maintien de la canule permanente (3) dans une position permanente, ainsi qu'un coulissement en arrière du premier coulisseau (4) et ainsi un retrait de la canule insérable (2) hors de la zone terminale distale de la canule permanente (3) dans une position finale, en particulier des côtés (15, 16) différents de la deuxième section (14) agissant sur le premier et sur le deuxième coulisseau (4, 5).

8. Appareil de dosage (20) selon l'une quelconque des revendications 2 à 7, la canule insérable (2) et la canule permanente (3) étant disposées, dans la position initiale, essentiellement à l'intérieur d'un boîtier (7) et pouvant être sorties du boîtier (7) par une ouverture d'enfoncement (9) dans la surface de contact (8) pour l'enfoncement de la canule permanente (3).

9. Appareil de dosage (20) selon l'une quelconque des revendications précédentes, l'ouverture d'aspiration (26) pouvant être mise en communication fluidique avec l'espace intérieur du réservoir.

10. Appareil de dosage (20) selon l'une quelconque des revendications précédentes, le dispositif de transport (5) comprenant au moins une autre ouverture, en particulier une ouverture d'aspiration et/ou d'évacuation, laquelle est disposée décalée dans la direction longitudinale sur le cylindre, comprenant plusieurs réservoirs, chaque ouverture d'aspiration pouvant être mise en communication fluidique avec l'espace intérieur d'un réservoir séparé qui lui est attribué.
